# EUROPEAN PATENT SPECIFICATION

(11) **EP 0 841 324 B1**
(45) Date of publication and mention of the grant of the patent: **10.09.2003**
(21) Application number: 97118621.8
(22) Date of filing: 27.10.1997
(51) Int. Cl.: C07C 337/02

(54) **A proces for preparing methyl dithiocarbazinate by reacting methyl bromide with the reaction product of carbon disulfide, hydrazine and an adjunct base**
Verfahren zur Herstellung von Methyldithiocabazinat durch Umsetzen von Methylbromid mit dem Reaktionsprodukt von Schwefelkohlenstoff, Hydrazin und einer Zusatzbase
Procédé pour la préparation de dithiocarbazinate de méthyle par réaction entre le bromure de méthyle et le produit de la réaction entre le disulphure de carbone, hydrazine et un base adjoint

(30) Priority: 07.11.1996 US 743763
(43) Date of publication of application: 13.05.1998
(73) Proprietor: Bayer Corporation, Pittsburgh, PA 15205-9741 (US)
(72) Inventor: Jackman, Dennis E., Prarie Village, KS 66208 (US); Erdman, David T., 51062 Köln (DE); Newallis, Peter E., Leawood, KS 66206 (US); Wasleski, Daniel M., Blue Springs, MO 64014 (US); Desai, Vijay C., Shawnee, KS 66216 (US); Macke, Jeffrey D., Kansas City, MO 64155 (US); Prasad, Vidyanatha A., Leawood, KS 66224 (US)
(74) Representative: Schumacher, Günter, Dr.

(56) References cited:
- EP-A- 0 366 300
- DE-C- 3 709 414
- GB-A- 1 274 521
- US-A- 3 284 482
- US-A- 4 696 938
- S.K. SENGUPTA, ET AL.: "Preparation and characterisation of complexes of bis-pi-cyclopentadienyl and bis-pi-indenyl molybdenum(VI) oxodichloride with thio-Schiff bases derived from S-methyldithiocarbazate" SYNTHESIS AND REACTIVITY IN INORGANIC AND METALLORGANIC CHEMISTRY, vol. 12, no. 3, 1982, pages 297-307, XP002173191 Marcel Dekker Inc., New York, US ISSN: 0094-5714
- R.S. DRAGO, ET AL.: "Synthesis, characterisation, and chemical reactivity of soluble bimetallomers" JOURNAL OF THE AMERICAN CHEMICAL SOCIETY, vol. 105, no. 8, 20 April 1983 (1983-04-20), pages 2287-2296, XP002173192 American Chemical Society, Washington, DC, US ISSN: 0002-7863
- A. AL KUBAISI, ET AL.: "Nickel(II) and palladium(II) chelates of dehydroacetic acid Schiff bases derived from thiosemicarbazide and hydrazinecarbodithioate" CANADIAN JOURNAL OF CHEMISTRY, vol. 72, no. 8, August 1994 (1994-08), pages 1785-1788, XP002173193 National Research Council, Ottawa, CA ISSN: 0008-4042

## Description

The present invention relates to an improved process for preparing methyl dithiocarbazinate. More specifically, the present invention relates to an improved process for preparing methyl dithiocarbazinate by reacting carbon disulfide, hydrazine and an adjunct base.

### Brief Description of the Prior Art:

Illustrative of the prior art of preparing methyl dithiocarbazinate are the following: Audrieth et al., J. Organic Chem., Vol. 19, pp. 733-741 (1954) discloses a process for preparing methyl dithiocarbazinate and converting it to thiocarbohydrazide. The process comprises a dropwise addition of 1.04 moles of carbon disulfide to 1.18 moles of potassium hydroxide and 1.1 moles of 85% hydrazine in 200 ml. of ethanol, in an ice bath. A heavy yellow oil containing potassium dithiocarbazinate separates during the addition. The resulting mixture is stirred, chilled and two volumes of ether are added to cause separation of more of the desired product (potassium dithiocarbazinate).

The oily layer is separated from the ether-alcohol layer and filtered to remove a small amount of an unidentified solid which is formed. The clear yellow solution is then dissolved in 300 ml. of water. The resulting solution is cooled in an ice bath and 1.05 moles of methyl iodide is added in approximately 10 batches. The reaction vessel is shaken and cooled alternately after each such addition until the methyl iodide is consumed. The reaction mixture is allowed to stand for several hours, and shaken occasionally to permit complete reaction. The methyl dithiocarbazinate is collected and recrystallized from ethanol.

Methyl dithiocarbazinate (24.4 gm, 0.2 mole) was dissolved in 200 ml. of absolute ethanol and 18 ml. (0.3 mole of hydrazine) of 85% hydrazine hydrate was added. The resulting solution was refluxed until no more solid thiocarbohydrazide precipitated (about 45 minutes). To remove a small amount of formed 3-hydrazino-4-amino-5-mercapto-1,2,4-triazole, the reaction mixture was chilled, and the resulting solid product was collected and recrystallized from acidified water (with a few drops of hydrochloric acid).

Le, U.S. Patent 4,696,938 discloses a process for preparing and using methyl hydrazinecarbodithioate as an intermediate in the preparation of 6-aryl-pyridine thiosemicarbazones. Methyl dithiocarbazinate is prepared as follows: Hydrazine hydrate (150 g) is added to a cooled (O°C) solution of potassium hydroxide in water (240 ml.) and 2-propanol (200 ml.). Pre-cooled carbon disulfide (182 ml.) is then added dropwise to the stirred reaction mixture, while maintaining the internal temperature below 10°C. After the addition is complete, stirring is continued for a further one hour. Cooled methyl iodide (426 g) is added dropwise for 1 ½ hour. The resulting white precipitate is collected via filtration and washed with cooled water. The crude product is recrystallized from methylene chloride.

To form 6-aryl-pyridine thiosemicarbazone, methyl dithiocarbazinate is reacted in a suitable solvent such as alcohol with the product of 6-aryl-2-alkylpyridine treated with selenium dioxide in a suitable ethereal solvent such as tetrahydrofuran or 1,4-dioxane.

S. Losanitch, J. Chem. Soc., Vol. 119, pp. 763-765 (1921) discloses a process for preparing methyl dithiocarbazinate by first obtaining ammonium dithiocarbazinate and reacting it with methyl iodide. The ammonium dithiocarbazinate is obtained when a solution of hydrazine hydrate in alcohol, containing a large excess of ammonia, is slowly treated with cooling with the corresponding quantity of carbon disulfide. The methyl dithiocarbazinate is formed by treating the ammonium salt in a dilute alcohol solution with methyl iodide.

Sandstrom et al, Arkiv For Kemi, 4(1952) 297, discloses a process for preparing ethyl dithiocarbazinate by decomposing diethyltrithiocarbonate with hydrazine. The process involves the separation of hydrazinium dithiocarbazinate from an ethanol-water mixture and the reaction of the hydrazinium dithiocarbazinate with ethyl bromide in an ethanol-water mixture.

U.S. Patent 3,284,482 discloses a process for preparing chlorobenzyl esters of dithiocarbazinic add as follows: To a solution comprising 85% hydrazine, 25% sodium hydroxide and 300 ml. of water is added carbon disulfide, dropwise at 10 to 15°C over 20 minutes. External cooling is removed and the reaction mixture is stirred for an hour at 25 to 30°C. Then, trichlorobenzyl chloride is added in one portion to the reaction mixture which is stirred for 24 hours at 25 to 30°C to produce the corresponding trichlorobenzyl dithiocarbazinate. The product is then extracted with ethyl ether. The ether solution is washed with water until it becomes neutral, is dried over sodium sulfate, and the ether is removed in vacuo.

British Patent Specification 1,274,521 discloses dithiocarbazinic ester derivatives by reacting dithiocarbazinic acid esters with an oxo compound. The dithiocarbazinic add is prepared by reacting hydrazine hydrate with carbon disulfide in alcohol medium in the presence of potassium hydroxide, ammonia or excess hydrazine hydrate.

After isolation, the dithiocarbazinic add salt is converted into an ester by an alkylating or aralkylating step. This step is carried out in water, a mixture of water and alcohol or in alcohol. Alternately, the ester can be prepared by a method wherein the reaction is carried out in a single reactor. The alkylating or aralkylating agent is added to the dithiocarbazinic acid salt solution prepared by the above method. The alkylating or aralkylating agents disclosed by the patent are: dimethyl sulfate, diethyl sulfate, allyl chloride, *n*-butyl iodide, *n*-octyl ester, *n*-dodecyl bromide, cetyl bromide, benzyl chloride, *p*-chlorobenzyl chloride, *p*-isopropylbenzyl bromide, *p*-*n*-butylbenzyl bromide, and alphamethylbenzyl chloride.

As would be realized from the foregoing, there is a need for an economical process, i.e., a more facile and cost efficient process for preparing methyl dithiocarbazinate. In particular, there is a need for a more economic commercial process for preparing methyl dithiocarbazinate. By the present invention, there is provided such an improved process for preparing methyl dithiocarbazinate.

### SUMMARY OF THE INVENTION

Surprisingly, it has been found that methyl dithiocarbazinate can be prepared by a facile and cost effective process. This improved process comprises reacting hydrazine, carbon disulfide and a base in an aqueous reaction medium, in an effective ratio to form a dithiocarbazinate salt, followed by reacting (methylating) the dithiocarbazinate salt with methyl bromide.

In the present embodiment of the invention, carbon disulfide, hydrazine, and an inorganic base are reacted in water as the reaction vehicle. The resulting reaction mixture comprising dithiocarbazinate salt is reacted with methyl bromide to produce high yields of methyl dithiocarbazinate. Preferably, the reaction medium comprises water as the reaction vehicle and, optionally, a hydrocarbon solvent such as toluene.

In contrast, the prior art processes generally involved the reaction of carbon disulfide and hydrazine in a reaction medium containing alcohols; separation of the resulting dithiocarbazinate; the use of relatively more expensive or more intractable and sometimes commercially cost prohibitive alkylating agents such as methyl iodide; and catalysts such as sodium iodide and/or recrystallization of the resulting methyl dithiocarbazinate.

It is a distinct feature of the invention that by using the process of the invention one can eliminate the use of undesirable methylating agents such as methyl iodide, undesirable solvents such as alcohols, and expensive catalysts such as sodium iodide. It is a distinct feature of the invention that the process requires short reaction times, requires no isolation of intermediate dithiocarbazinate salts, and no recrystallization of the final methyl dithiocarbazinate. Consonantly, the process of the invention fills a long felt but unmet need of using a facile process for preparing methyl dithiocarbazinate by using readily available and relatively less expensive reactants and solvents, and processing techniques.

In the practice of the invention, methyl dithiocarbazinate can be used as an intermediate compound in the preparation of other chemicals such as thiadiazoles.

### DETAILED DESCRIPTION OF THE INVENTION

As afore-stated, the claimed invention relates to a process for preparing methyl dithiocarbazinate (MDTC) by: (I) reacting carbon disulfide with hydrazine and a base in an aqueous reaction medium and in an effective ratio to form a dithiocarbazinate salt followed by (II) methylating the dithiocarbazinate salt of (I) with methyl bromide. The process can be represented by reactions (I) and (II), as follows: 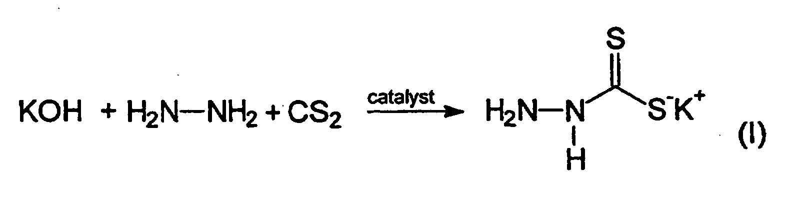 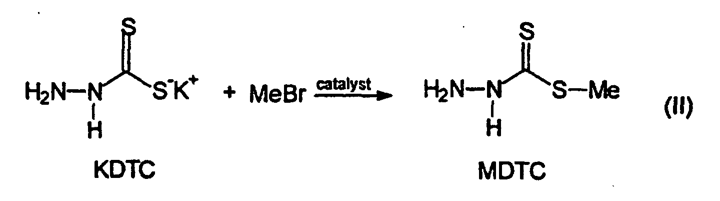 Illustratively, hydrazine (usually in the form of hydrazine hydrate), carbon disulfide and an inorganic base are reacted in a mole ratio of about 1:1.2:1 to 1.2:1:2 and, preferably, 1:1:1. For example, carbon disulfide and the inorganic base are added to hydrazine simultaneously and, preferably, dropwise. A phase transfer catalyst can be employed herein. Alternately, half of the carbon disulfide and half of the inorganic base can be first charged to the hydrazine, and then the other halves of carbon disulfide and the inorganic base are added.

The reaction can be conducted in a pH range that does not adversely affect the reaction. Typically, the reaction ran be conducted in a pH range of about 8 to 14 and preferably 9 to 14. The reaction temperature can be about 0 to 35°C and preferably 5 to 25°C. Time of the reaction can be about 1 to 4 hours and preferably 1 to 2 hours.

As the base, one can employ inorganic and nitrogenous bases as described hereunder. The base, in contradistinction with hydrazine, is described herein as an adjunct base. The inorganic base is preferably potassium hydroxide and more preferably powdered potassium hydroxide. It is believed without being bound to any particular theory that, other than potassium hydroxide, one can use other inorganic bases that can affect sufficient ion exchange with the subject hydrazinium ion. Illustrative of these other inorganic bases would be sodium hydroxide and lithium hydroxide. As the nitrogenous base, one can employ a nitrogenous compound such as ammonia, ammonium hydroxide and certain amines. The amines can be ethanolamine (which is preferred), di-*n*-propylamine, diisopropylamine, di-*n*-butylamine, *t*-butylamine, dimethyl benzylamine and ethylmethyl pyridine. ,

The aqueous reaction medium typically contains water and optionally a non-aqueous solvent. The mole ratio of water to carbon disulfide can be about 2 to 10 and preferably 2 to 5 moles. A non-limiting example of the solvent can be hydrocarbon solvent such as an aromatic solvent selected from the group consisting of: xylene, cumene, benzene, toluene, ethyl benzene and mesitylene or aliphatic solvent selected from the group consisting of: pentane, hexane, cyclohexane and heptane. Typically, the solvent is an aromatic solvent which is preferably toluene. The solvent can be employed in a mole ratio of 0.1 to 3 and preferably 0.15 to 2 moles per mole of carbon disulfide. Unlike many of the prior art processes, the reaction medium of this process need not contain aprotic solvents. The reaction medium of this process consists essentially of water, and optionally a non-alcoholic solvent.

The resulting product comprising the dithiocarbazinate salt is reacted with methyl bromide. The mole ratio of methyl bromide to the dithiocarbazinate salt can be from 1.5 to 1.02:1, and preferably 1.05:1. The methylation reaction temperature can be from about 0 to 35°C, over a period of about 0.5 to 3 hours and preferably 1 to 2 hours. The water, and optionally the solvent, and the catalyst employed in reaction (II) are essentially the same as described in reaction (I). The mole ratio of the water to the dithiocarbazinate salt can be from about 1 to 3:1 and preferably 1:1. The mole ratio of the solvent to the dithiocarbazinate salt can be from about 1.3 to 0.17:1 and preferably 0.5:1. The catalyst can be used in amounts of about 0.5 mole % to 10% preferably 0.5% to 1% per mole of dithiocarbazinate salt.

While the methylation reaction (II) ran be conducted in another vessel, it is typically conducted in the same reaction vessel as used in the preparation of the dithiocarbazinate salt. It is a distinct feature of the invention that the methylation reaction can be conducted without isolating the dithiocarbazinate salts of reaction (I). In this embodiment of the invention, the methylating reaction (II) consists essentially of reacting the reaction product of reaction (I) with methyl bromide. Alternately, the dithiocarbazinate salt can be isolated by filtration and subsequently reacted with methyl bromide. Illustratively, the dithiocarbazinate salt can be filtered, slurried in a reaction medium such as described above, and then reacted with methyl bromide. The reaction conditions would be essentially the same as described above.

It is also a distinct feature of the invention that the methylating reaction (II) can be conducted without the use of expensive reaction catalysts such as sodium iodide. In this regard, the methylating step can be said to consist essentially of reacting the reaction product of (I) with methyl bromide.

The resulting product containing methyl dithiocarbazinate can be isolated by any convenient means such as filtering or centrifuging. The methyl dithiocarbazinate can be collected on a vacuum filter and washed with water to remove impurities such as potassium bromide salt. The resulting wet cake can be used as such or dried by any means that is effective to provide the requisite drying without causing its decomposition. Illustratively, the product can be dried at temperatures that do not cause decomposition of the products. More specifically, the product can be dried in a vacuum oven, using a nitrogen sparge at a temperature of about 30° to 40°C. Generally, the methyl dithiocarbazinate purity can be up to about 95 percent with variation attributable to washing and/or drying steps. As would be realized from the above, methyl dithiocarbazinate can be obtained without recrystallization of the reaction product of reaction (II). Therefore, methyl dithiocarbazinate can be prepared by a process consisting essentially of reacting carbon disulfide, hydrazine and a base in an aqueous medium, in an effective ratio to form a dithiocarbazinate salt, followed by methylating the dithiocarbazinate salt with methyl bromide, and removing the bromide salt.

A catalyst, such as a phase-transfer catalyst can be employed in the reaction(s). An example of the catalyst is tris-[2-(2-methylethoxy) ethyl] amine (TDA-1), *N*-benzyltrimethylammonium hydroxide (available from Aldrich Chemical Co.), *N*-methylimidazole, dimethylaminopyridine, 1,4-diazabicyclo-(2,2,2)-octane and diethylene glycol. The mole ratio of the catalyst can be 0 to 300 mmoles and preferably 0 to 2 mmoles per mole of carbon disulfide.

The following is a convenient, but non-limiting, description of the process for preparing the methyl dithiocarbazinate in accordance with this invention. Carbon disulfide is slowly added (with cooling) to a mixture of toluene, water, TDA-1, and hydrazine monohydrate at a temperature below 25°C. When the pH of the resulting slurry is stabilized at 9 to 10, aqueous potassium hydroxide is added to the mixture and the reaction mixture is stirred until the pH drops back to about 9. Three more equal portions of carbon disulfide and potassium hydroxide are added using the same protocol as described above. After the completion of the addition of these portions, the reaction mixture is stirred for 90 minutes, then methyl bromide gas is bubbled into the mixture over one hour. Typically, a white slurry is produced, stirred for about 30 minutes at about 25°C, cooled to about 0 to 5°C, and filtered. The filtered product which is usually in the form of a white powder, analyses as methyl dithiocarbazinate with a yield in the range of about 60 to 80 percent. The active ingredient (Al) is in the range of 65 to 95 percent. The major impurities are water and potassium bromide.

As would be seen from the foregoing, the process of the invention is well suited to commercial applications because it employs readily available reagents and processing techniques. Illustratively, the process can be carried out under reaction conditions which do not produce environmentally unacceptable by-products, reduce yield, affect quality of the end products or adversely impact production operations.

These and other aspects of the invention are further illustrated but are not intended to be limited by the following examples in which all parts and percentages are by weight unless otherwise specified.

### EXAMPLES

### Example 1:

### Preparation of Methyl Dithiocarbazinate (MDTC) Using Aqueous KOH as Base

In a properly equipped reaction vessel, carbon disulfide (19 g, 0.25 mole) was slowly added (with cooling) to a mixture of toluene (43.25 g, 0.46 moles), water (72 g, 4 moles), TDA-1 (2.02 g, 0.0063 moles), and hydrazine monohydrate (50 g, 1.0 mole) at <25°C. When the pH of the resulting slurry stabilized at pH 9, aqueous potassium hydroxide (43%, 32.6 g., 0.25 mole) was added and the solution was stirred until the pH dropped to 9. Three more equal portions of carbon disulfide (19 g, 0.25 moles) and potassium hydroxide (43%, 32.6 g, 0.25 moles) were added using the same protocol. After the completion of the addition of these portions, the reaction mixture was stirred for 90 minutes.

Methyl bromide gas (104.4 g, 1.1 moles) was bubbled into the yellow mixture over one hour. The resulting white slurry was stirred for an additional 30 minutes at 25°C, cooled to 5°C and filtered. Methyl dithiocarbazinate was isolated as a white powder at 77% yield. The major impurities were water and potassium bromide.

### Example 2:

### Preparation of Methyl Dithiocarbazinate (MDTC) Using Solid KOH

In a properly equipped reaction vessel, carbon disulfide (76 g, 1.0 mole) was slowly added (with cooling) to a mixture of toluene (43.25 g, 0.46 moles), water (72 g, 4 moles), TDA-1 (1.98 g, 0.0061 moles), freshly ground solid KOH (56 g, 1.0 moles) and hydrazine monohydrate (50 g, 1.0 moles) at <25 °C. The reaction mixture was stirred for 2 hours, then methyl bromide gas (104.4 g, 1.1 moles) was bubbled into the resulting yellow mixture over one hour. The mixture turned into a white slurry which was stirred for 30 minutes at 25°C, then cooled to 5°C and filtered. Methyl dithiocarbazinate was isolated as a white powder at 72% yield. The major impurities were water and potassium bromide.

### Example 3:

### Preparation of Methyl Dithiocarbazinate (MDTC) Using Aqueous NaOH

The ingredients and method of preparation were essentially the same as described in Example 1 except that 50% NaOH instead of 43% KOH was employed. The yield of MDTC was 70%. The major impurities were water and sodium bromide.

### Example 4:

### Preparation of Methyl Dithiocarbazinate (MDTC) Using Ammonium Hydroxide

In a properly equipped reaction vessel, hydrazine hydrate (75.0 g, 1.50 mol.) was slowly added (with cooling) to a mixture of ammonium hydroxide (87.1 g, 1.54 mol.) and water (75 g, 4.16 moles) at <5°C. Carbon disulfide (114.0 g, 1.50 mol.) was then added (1.0 hour) to the mixture while maintaining the temperature at 5°C. After the mixture had been stirred for about 1 hour at the same temperature, toluene (43.25 g, 0.46 moles) and diethylene glycol (4.47 g, 0.042 moles) were charged, and methyl bromide (156.7 g, 1.65 mol.) was introduced over a period of 2 hours at 5°C. The mixture was agitated for 1 hour at 25°C, cooled to 0 to 5°C, and the solids were filtered. The solids were washed with ice-cold water (3 x 25 ml.), and then dried (overnight at 40°C/20 mm) to yield 177.1 g (77.6%) of white crystals. The mother liquor contained an additional 3 to 5% net yield of MDTC.

### Example 5:

### Preparation of Methyl Dithiocarbazinate (MDTC) Using t-Butylamine

In a properly equipped reaction vessel, hydrazine hydrate (75.0 g, 1.50 mol.) was slowly added (with cooling) to a mixture of *t*-butylamine (109.7 g, 1.50 mol.) and water (75 g, 4.2 moles) and toluene (43.25g, 0.469 moles) at <5°C. Carbon disulfide (114.0 g, 1.50 mol.) then was added (1 hour) to the mixture while maintaining the temperature at 5°C. After the mixture had stirred for about 1 hour at the same temperature, methyl bromide (156.7 g, 1.65 mol.) was introduced over a period of 2 hours at 5°C. The mixture was agitated for 1 hour at 25°C, cooled to 0 to 5°C, and the solids were filtered. The solids were washed with ice-cold water (3 x 25 ml.), and then dried (overnight at 40°C/20 mm) to yield 156.4 g, (81.0%) of white crystals. The mother liquor contained an additional 3 to 5% net yield of MDTC.

### Example 6:

### Preparation of Methyl Dithiocarbazinate (MDTC) Using KOH

In a properly equipped reaction vessel (four neck, 1000 ml.) hydrazine hydrate (50.0 g,1.0 mol.) was slowly added (with cooling) to a mixture of toluene (86.5 g, 0.94 moles), water (72 g, 4.0 moles) and TDA-1 (2.0 g, 0.00062 moles) at <25°C). Carbon disulfide (12.7 g, 0.167 mol.) was added dropwise over a 10 minute period while maintaining the temperature at 25°C, followed by the addition of KOH (21.75 g, 0.167 mol) over a 10 minute period, while maintaining the temperature at 25°C. The incremental addition of carbon disulfide and KOH was repeated five or more times, including the cool periods. Methyl bromide (104.5 g, 1.1 moles) was then added over a period of 2 hours at 25°C. The reaction mixture was cooled to 0 to 5°C, and filtered under vacuum. The resultant product was then washed with cold water and then dried in a vacuum oven (40°C/20 mm) to yield 92.8 g (76.1%) of MDTC. The mother liquor contained an additional 3 to 5% net yield of MDTC.

## Claims

1. A process for preparing methyl dithiocarbazinate by reacting carbon disulfide, hydrazine and an adjunct base in an aqueous medium, in an effective ratio to form a dithiocarbazinate salt, followed by methylating the dithiocarbazinate salt with methyl bromide.

2. The process of Claim 1 wherein the adjunct base is an inorganic base which is potassium hydroxide or sodium hydroxide.

3. The process of Claim 1 wherein the adjunct base is a nitrogenous base selected from the group consisting of ammonia, ammonium hydroxide and an amine.

4. The process according to Claim 1 wherein the reaction is in the presence of a catalyst.

5. The process of Claim 1 wherein the mole ratio of hydrazine to carbon disulfide to the base is 1.2:1:2 to 1:1.2:1.

6. The process of Claim 5, wherein the mole ratio is 1:1:1.

7. The process of Claim 1, wherein the mole ratio of methyl bromide to the dithiocarbazinate salt is 1.5 to 1.02:1.

8. The process according to Claim 1, wherein the aqueous medium comprises . water or water and a hydrocarbon solvent.

9. The process according to Claim 8, wherein the aqueous medium consists of water and a hydrocarbon solvent.

10. The process according to Claim 1 or 8, wherein the reaction is conducted in a pH range of 8 to 14.

## Patentansprüche

1. Verfahren zur Herstellung von Methyldithiocarbazinat durch Umsetzen von Schwefelkohlenstoff, Hydrazin und einer zusätzlichen Base in einem wässrigen Medium in einem effektiven Verhältnis zur Bildung eines Dithiocarbazinat-Salzes und anschließende Methylierung des Dithiocarbazinat-Salzes mit Methylbromid.

2. Verfahren gemäß Anspruch 1, wobei die zusätzliche Base eine anorganische Base ist, bei der es sich um Kaliumhydroxid oder Natriumhydroxid handelt.

3. Verfahren gemäß Anspruch 1, wobei die zusätzliche Base eine Stickstoffbase ist, die aus der Gruppe ausgewählt ist, die aus Ammoniak, Ammoniumhydroxid und einem Amin besteht.

4. Verfahren gemäß Anspruch 1, wobei die Reaktion in Gegenwart eines Katalysators durchgeführt wird.

5. Verfahren gemäß Anspruch 1, wobei das Stoffmengenverhältnis von Hydrazin zu Schwefelkohlenstoff zur Base 1,2:1:2 bis 1:1,2:1 beträgt.

6. Verfahren gemäß Anspruch 5, wobei das Stoffmengenverhältnis 1:1:1 beträgt.

7. Verfahren gemäß Anspruch 1, wobei das Stoffmengenverhältnis von Methylbromid zu dem Dithiocarbazinat-Salz 1,5 bis 1,02 zu 1 beträgt.

8. Verfahren gemäß Anspruch 1, wobei das wässrige Medium Wasser oder Wasser und ein Kohlenwasserstoff-Lösungsmittel umfasst.

9. Verfahren gemäß Anspruch 8, wobei das wässrige Medium aus Wasser und einem Kohlenwasserstoff-Lösungsmittel besteht.

10. Verfahren gemäß Anspruch 1 oder 8, wobei die Reaktion in einem pH-Bereich von 8 bis 14 durchgeführt wird.

## Revendications

1. Procédé pour préparer du dithiocarbazinate de méthyle par réaction de disulfure de carbone, d'hydrazine et d'une base adjointe dans un milieu aqueux, dans un rapport efficace pour former un sel de dithiocarbazinate, puis méthylation du sel de dithiocarbazinate avec le bromure de méthyle.

2. Procédé selon la revendication 1, dans lequel la base adjointe est une base inorganique qui est l'hydroxyde de potassium ou l'hydroxyde de sodium.

3. Procédé selon la revendication 1, dans lequel la base adjointe est une base azotée choisie parmi le groupe constitué d'ammoniac, d'hydroxyde d'ammonium et d'une amine.

4. Procédé selon la revendication 1, dans lequel la réaction est effectuée en présence d'un catalyseur.

5. Procédé selon la revendication 1, dans lequel le rapport molaire de l'hydrazine au disulfure de carbone à la base est de 1,2:1:2 à 1:1,2:1.

6. Procédé selon la revendication 5, dans lequel le rapport molaire est 1:1:1.

7. Procédé selon la revendication 1, dans lequel le rapport molaire du bromure de méthyle au sel de dithiocarbazinate est 1,5 à 1,02:1.

8. Procédé selon la revendication 1, dans lequel le milieu aqueux comprend de l'eau ou de l'eau et un solvant hydrocarboné.

9. Procédé selon la revendication 8, dans lequel le milieu aqueux consiste en eau et en un solvant hydrocarboné.

10. Procédé selon la revendication 1 ou 8, dans lequel la réaction est effectuée dans un intervalle de pH de 8 à 14.
